# EUROPÄISCHE PATENTSCHRIFT

(11) **EP 0 501 088 B1**
(45) Veröffentlichungstag und Bekanntmachung des Hinweises auf die Patenterteilung: **11.10.1995**
(21) Anmeldenummer: 91710056.2
(22) Anmeldetag: 24.12.1991
(51) Int. Cl.: A61B 1/04

(54) **Endoskop mit proximal ankuppelbarer Kamera**
Endoscope with proximal coupled camera
Endoscope avec caméra accouplée proximale

(30) Priorität: 21.02.1991 DE 4105326
(43) Veröffentlichungstag der Anmeldung: 02.09.1992
(73) Patentinhaber: Richard Wolf GmbH, 75438 Knittlingen (DE)
(72) Erfinder: Bonnet, Ludwig, W-7134 Knittlingen (DE)
(74) Vertreter: Wilcken, Thomas, Dipl.-Ing.

(56) Entgegenhaltungen:
- EP-A- 0 058 020
- US-A- 4 248 213
- US-A- 4 781 448
- US-A- 4 851 866

## Beschreibung

Zur Übertragung von Bildern aus Körperhöhlen und/oder zur Befunddokumentation werden Kameras, insbesondere Videokameras, verwendet, die über ihr Objektiv mit dem Okulartrichter des Endoskops lösbar, im übrigen aber starr verbindbar sind.

Verbindungen bzw. Schnellkupplungen der erwähnten Art sind bekannt aus den DE-U 18 40 515 und DE-U 79 18 414 sowie der DE-A 34 29 945 und der DE-B 27 57 358. Diese bekannten Ausführungen weisen den Nachteil auf, daß durch die feste Verbindung zwischen dem Okulartrichter und der Kamera ein Verdrehen der Kamera auf dem Okulartrichter nicht ohne weiteres möglich ist, wodurch die Handhabung der gesamten Einheit, bestehend aus Endoskop bzw. Endoskopoptik, Objektiv mit Schnellkupplung und Kamera, in manchen Fällen sehr ungünstig ist, insbesondere dann, wenn bei unveränderter Beobachtungsposition der Angriffsort des Behandlungsinstrumentes an einer ausgewählten Gewebestelle verändert werden soll, um dadurch beispielsweise eine Blutung stillen, eine Koagulation oder einen Gewebeschnitt vornehmen zu können.

Ein geringfügiges bzw. beschränktes Verdrehen des verwendeten Instruments, wie beispielsweise eine Koagulation-Schneid-Schlinge, ist zwar bei starrer Adaption einer Kamera am Okulartrichter einer Endoskopoptik grundsätzlich möglich, weist jedoch den Nachteil der damit gleichzeitig einhergehenden Bilddrehung auf, wodurch das mittels eines Monitors dargestellte endoskopische Bild sehr unruhig auf den Betrachter wirkt und dieser unter Umständen dadurch die Orientierung verliert.

Durch die aus vorstehend genanntem Grunde vorzunehmende Korrektur der Bildlage wird der Operateur genötigt, den Arbeitsablauf zu unterbrechen, um die erforderliche Korrektur vornehmen zu können, wodurch zum einen für den Eingriff ein größerer Zeitaufwand erforderlich ist als dies für die Ausführung des Eingriffs selbst erforderlich wäre und andererseits durch die Unterbrechung und Wiederaufnahme des Eingriffes auch der geistige Arbeitsablauf des Operateurs nachteilig beeinträchtigt zumindest jedoch gestört wird.

Ein Endoskop mit mechanischer Kompensation von Bilddrehungen ist aus US-A-4 248 213 bekannt. Die bekannte Vorrichtung weist einen Adapter auf, der eine gewichts Kompensiert aufgehängte Kamera dreht.

Die Aufgabe der Erfindung besteht daher darin, diese Nachteile zu vermeiden, dem Operateur die Durchführung eines Eingriffes in der Körperhöhle zu erleichtern, die Anforderung an die Konzentration des Operateurs zu reduzieren und den endoskopischen Eingriff sicher und schnell durchführen zu können. Die Aufgabe besteht insbesondere darin, die Kupplung zwischen Kameraobjektiv und Endoskop konstruktiv so auszubilden, daß mögliche Bilddrehungen automatisch ausgeglichen bzw. verhindert werden, ohne hierzu elektronische Meß- und Stelleinrichtungen anwenden zu müssen.

Diese Aufgabe wird durch den Anspruch 1 gelöst. Die Unteransprüche stellen spezielle Lösungen der erfindungsgemäßen Aufgabe dar.

Die Erfindung wird nachstehend anhand der Zeichnung erläutert. Es zeigen:

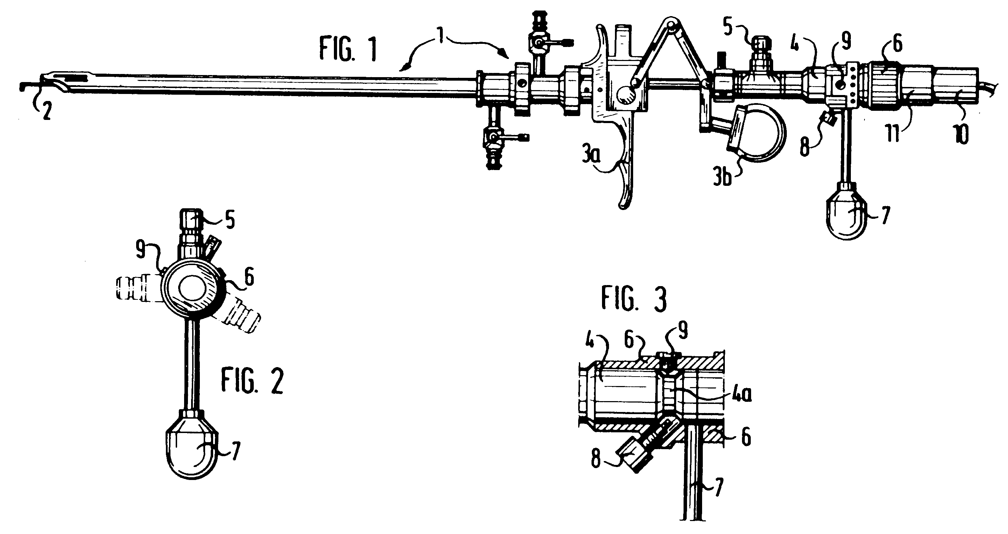

- Figur 1: die Seitenansicht eines Resektoskops mit angekuppeltem Objektiv einer Videokamera,
- Figur 2: die teilweise Ansicht auf das proximale Ende des Objektives nach Figur 1 und
- Figur 3: einen vergrößerten Längsschnitt durch das Kameraobjektiv und benachbarte Teile des Endoskops nach Figur 1.

Das Endoskop in Form eines Resektoskops 1 ist am offenen, distalen Schaftende mit einer HF-Schneidschlinge 2 versehen, die durch eine Handhabe 3a, 3b zur Durchführung eines Längsschnittes, z.B. zum Abtragen von Prostatagewebe, axial verschiebbar ist.

Am proximalen Ende des mit dem Außenschaft lösbar gekuppelten Arbeitseinsatzes befindet sich der Okulartrichter 4 der lösbar im Arbeitseinsatz festgelegten Endoskopobtik 5, auf den das distale Ende des Objektives 6 einer Kamera aufgeschoben und durch Kugelrastelemente 9 drehbar fixiert ist. Diese Fixierung bzw. Kupplung ist durch eine in die Ringnut 4a des Okulartrichters 4 eingreifende Schraube 8 gegen unbeabsichtigtes Lösen gesichert. Diese Schraube 8 ist in der Fassung des Objektives 6 der Videokamera, von der nur ihr Kamerakopf 10 gezeigt ist, verschraubbar und mit einer Anschlagschulter versehen, um zu verhindern, daß die Schraube 8 gegen den Boden der Ringnut Zur Anlage kommt und dadurch die Verdrehung des Objektivs 6 auf dem Okulartrichter 4 behindert. Der Kamerakopf ist in das proximale Ende 11 des Objektives 6 eingeschraubt.

Der Schwerpunkt des verdrehbar an dem Okulartrichter 4 gelagerten Kameraobjetivs 6 ist exzentrisch zur Drehachse verlagert. Dies wird nach dem Ausführungsbeispiel dadurch erreicht, daß mit dem Objektiv 6 ein nach unten gerichtetes Gewichtspendel 7 starr verbunden ist. Dieses Pendel 7 kann bei Bedarf durch ein jeweils anderes Pendel mit abweichendem Gewicht ersetzt werden. In jedem Fall wird die Lage des Objektives 6 bzw. die Aufnahmeposition der Videokamera bei erforderlich werdender Verdrehung des Endoskops 1 beibehalten, und zwar durch Auspendeln der Kamera bzw. des Kamerakopfes am Endoskop. Es kann sich daher die Drehbewegung des Endoskops nicht auf das auf einen Monitor dargestellte endoskopische Bild auswirken bzw. es wird ein Mitbewegen des Objektives 6 mit der Verdrehung des Endoskops durch das Pendel rückgängig gemacht bzw. von vornherein zumindest weitgehend vermieden.

Es ist auch möglich, das Pendel 7 mit dem Oberende in die Ringnut 4a des Okulartrichters 4 eingreifen zu lassen, wodurch die Verbindung des freiverdrehbaren Objektivs mit dem Okulartrichter 4 sicher ist und die Sicherungsschraube 8 entfallen kann.

Eine exzentrische Verlagerung des Kamera- bzw. Objektivschwerpunktes ist auch dadurch möglich, daß das Einzelpendel 7 durch zwei im Winkel schräg nach unten divergierende, unmittelbar seitlich an der Objektivfassung angeordnete Stabilisatoren ersetzt wird.

Im übrigen brauchen keine gesonderten Pendel vorgesehen zu werden, wenn der Schwerpunkt der an das Endoskop angeschlossenen Kamerateile so zur Drehachse versetzt angeordnet werden kann, daß allein schon durch diese Maßnahme das Kameraobjektiv unabhängig von Verdrehungen des Endoskopes ausgependelt in seiner Position gehalten wird.

## Patentansprüche

1. Endoskop (1) mit einem proximal und lösbar angekuppelten Objektiv (6) einer Kamera, insbesondere einer Videokamera, wobei Mittel zum Ausgleich von Bilddrehungen vorgesehen sind, dadurch gekennzeichnet, daß das Kameraobjektiv (6) frei verdrehbar am Endoskop (1) gelagert ist und daß der Schwerpunkt der mit dem Endoskop verbundenen Kamerateile (6,7,10,11) so exzentrisch zur Drehachse verlagert ist, daß das Kameraobjektiv (6) bei Verdrehung des Endoskops (1) seine Lage durch Auspendeln beibehält.

2. Endoskop nach Anspruch 1, dadurch gekennzeichnet, daß die Exentrizität des erwähnten Schwerpunktes durch ein nach unten gerichtetes, mit dem Kameraobjektiv verbundenes Pendel (7) erreicht ist.

3. Endoskop nach Anspruch 1, dadurch gekennzeichnet, daß die Exzentrizität des erwähnten Schwerpunktes durch zwei im Winkel schräg nach unten gerichtete, seitlich an der Objektivfassung angeordnete Stabilisatoren erreicht ist.

4. Endoskop nach einem der Ansprüche 1 bis 3, dadurch gekennzeichnet, daß der Okulartrichter (4) des Endoskopes (1) mit einer Ringnut (4a) versehen ist, in die in der Kameraobjektivfassung befindliche Rastkugeln (9) eingreifen.

5. Endoskop nach Anspruch 4, dadurch gekennzeichnet, daß in die Ringnut (4a) des Okulartrichters (4) das Ende einer in der Objektivfassung angeordneten Schraube (8) oder das Ende des Pendels (7) eingreift.

## Claims

1. Endoscope (1) having a lens (6) of a camera, in particular a video camera, coupled proximally and releasably, wherein means for compensating image rotations are provided, characterised in that the camera lens (6) is mounted on the endoscope (1) to be twisted freely, and in that the centre of gravity of the camera parts (6, 7, 10, 11) connected to the endoscope is displaced eccentrically to the axis of rotation so that the camera lens (6) retains its position by oscillating when the endoscope (1) is twisted.

2. Endoscope according to claim 1, characterised in that the eccentricity of the centre of gravity mentioned is achieved by means of a pendulum (7) connected to the camera lens and pointing downwards.

3. Endoscope according to claim 1, characterised in that the eccentricity of the centre of gravity mentioned is achieved by means of two stabilisers arranged laterally on the lens mounting and pointing downwards at a diagonal angle.

4. Endoscope according to one of claims 1 to 3, characterised in that the eyepiece funnel (4) of endoscope (1) is provided with a circular groove (4a) into which locking balls (9) situated in the camera lens mounting engage.

5. Endoscope according to claim 4, characterised in that the end of a screw (8) arranged in the lens mounting or the end of pendulum (7) engages in the circular groove (4a) of the eyepiece funnel (4).

## Revendications

1. Endoscope (1) comportant un objectif (6), accouplé de façon amovible sur le côté proximal, d'une caméra, notamment d'une caméra vidéo, et dans lequel des moyens sont prévus pour compenser des rotations de l'image, caractérisé en ce que l'objectif (6) de la caméra est monté de manière à pouvoir tourner librement sur l'endoscope (1), et en ce que le centre de gravité des parties (6,7,10,11) de la caméra, qui sont reliées à l'endoscope, est déplacé dans une position excentrée par rapport à l'axe de rotation de telle sorte que l'objectif (6) de la caméra conserve sa position par un mouvement pendulaire, lors d'une rotation de l'endoscope (1).

2. Endoscope selon la revendication 1, caractérisé en ce que l'excentricité du centre de gravité mentionné est obtenue au moyen d'un pendule (7) dirigé vers le bas et relié à l'objectif de la caméra.

3. Endoscope selon la revendication 1, caractérisé en ce que l'excentricité du centre de gravité mentionné est obtenue au moyen de deux stabilisateurs, qui sont dirigés obliquement vers le bas sous un certain angle et sont disposés latéralement sur la monture de l'objectif.

4. Endoscope selon l'une des revendications 1 à 3, caractérisé en ce que l'entonnoir (4) de l'oculaire de l'endoscope (1) comporte une gorge annulaire (4a), dans laquelle s'engagent des billes d'encliquetage (9) situées dans la monture de l'objectif de la caméra.

5. Endoscope selon la revendication 4, caractérisé en ce que l'extrémité d'une vis (8), disposée dans la monture de l'objectif, ou l'extrémité du pendule (7) s'engage dans la gorge annulaire (4a) de l'entonnoir (4) de l'oculaire.
